## Europäisches Patentamt

## European Patent Office

## Office européen des brevets

(11) Veröffentlichungsnummer: **0 009 727**
**B1**

(12) **EUROPÄISCHE PATENTSCHRIFT**

(45) Veröffentlichungstag der Patentschrift:
**12.05.82**

(51) Int. Cl.³: **A 61 N 1/04**

(21) Anmeldenummer: **79103554.6**

(22) Anmeldetag: **20.09.79**

(54) Implantierbare Kohlenstoffelektrode.

(30) Priorität: **28.09.78 DE 2842318**

(43) Veröffentlichungstag der Anmeldung:
**16.04.80 Patentblatt 80/8**

(45) Bekanntmachung des Hinweises auf die Patenterteilung:
**12.05.82 Patentblatt 82/19**

(84) Benannte Vertragsstaaten:
**FR GB SE**

(56) Entgegenhaltungen:
**DE-A-2 810 004**
**FR-A-2 299 873**
**US-A-3 994 302**

(73) Patentinhaber: **SIEMENS AKTIENGESELLSCHAFT**
**Berlin und München, Postfach 22 02 61,**
**D-8000 München 22 (DE)**

(72) Erfinder· **Richter, Gerhard, Dr.,**
**Friedrich-Bauer-Strasse 18, D-8520 Erlangen (DE)**
Erfinder: **Weidlich, Erhard, Dr., Am Tennenbach 41,**
**D-8521 Spardorf (DE)**

Implantierbare Kohlenstoffelektrode

Die Erfindung betrifft eine implantierbare Kohlenstoffelektrode, insbesondere eine Reizelektrode.

Reizelektroden, beispielsweise für Herzschrittmacher, bestehen im allgemeinen aus einer isolierten Kabelzuleitung und einem Elektrodenkopf zur Übertragung der Stimulationsimpulse. Der elektrische Reizerfolg setzt die Erzeugung einer bestimmten elektrischen Feldstärke an der erregbaren Zellmembran voraus. Bei Reizleitungsstörungen im Herzen bewirkt eine Reizelektrode die Reizung einer Muskelfasermembran. Die Reizung breitet sich über die Faser aus und springt dann auf benachbarte Fasern über, bis schließlich der ganze Herzmuskel in den gereizten, d. h. kontrahierten Zustand überführt wird.

Zur Reizauslösung dient ein elektronischer Herzschrittmacher, der aus einem implantierbaren Elektronikteil mit einer Energieversorgungseinheit und einem Reizstromkreis mit einer Stimulationselektrode und einer indifferenten Elektrode besteht, Während des Impulses wird innerhalb von 0,5 bis 2 ms ein kleiner Kondensator über den Reizstromkreis teilweise entladen. In den Pausen zwischen den Impulsen wird der Kondensator aus der Energieversorgungseinheit, d. h. aus einer Batterie, wieder aufgeladen. Während des Impulses besteht im reizbaren Gewebe in der Nähe der Reizelektrode, die zur Auslösung des Reizes erforderliche Feldstärke.

Reizelektroden aus Metallen, wie beispielsweise Platin oder Elgiloy, bewirken eine langsame Degenerierung des an die Elektrode angrenzenden Gewebes. Innerhalb von 2 bis 4 Wochen bildet sich um die Elektrode eine nicht reizbare Bindegewebsschicht aus. Die Folge ist ein stetiger Anstieg der Reizschwelle. Die Elektrode wird also scheinbar größer und um an der Grenze zum reizbaren Gewebe wieder die erforderliche Feldstärke herzustellen, ist ein größerer Strom erforderlich und dementsprechend eine höhere Spannung. Damit steigen aber auch der ohmsche Verlust am Engewiderstand der Reizelektrode und die Polarisationsverluste durch elektrochemische Reaktionen an der Elektrodenoberfläche.

Es sind auch bereits Reizelektroden bekannt, bei denen ein metallischer Leiter mit einem Ionenaustauschermaterial in Form eines Polymeren mit aufgepfropften ionogenen Gruppen versehen ist (US-PS 3 994 302). Auf dem Ionenaustauschermaterial, das — zur Übertragung der elektrischen Signale — teilweise mit dem Körpergewebe in Berührung steht, ist dabei ein elektrisch isolierender und flüssigkeitsdichter Überzug angeordnet.

Als Ursache für die Abwehrreaktion des Körpers, die sich in der Ausbildung einer nicht reizbaren Gewebeschicht manifestiert, werden chemische und elektrochemische Prozesse angesehen, beispielsweise die elektrochemische Korrosion der Elektrode und die elektrolytische Zersetzung der Körperflüssigkeit sowie elektrolytische Reduktions- oder Oxidationsreaktionen oder mit diesen Reaktionen verbundene Verschiebungen des pH-Wertes.

Um derartigen Reaktionen mit den damit verbundenen Folgen zu entgehen, wird die Verwendung möglichst inerter Elektrodenmaterialien angestrebt, die weder korrodieren noch elektrokatalytische Reaktionen verursachen. So ist es bereits bekannt, als Elektrodenmaterial für Reizelektroden spektralreinen Graphit und Kohlenstoff zu verwenden.

Ferner ist es bereits bekannt, als Material für implantierbare Elektroden bzw. deren Elektrodenkopf Glaskohlenstoff (glasartigen Kohlenstoff) und Pyrokohlenstoff (pyrolytischen Kohlenstoff) zu verwenden (DE-OS 2 613 072 bzw. 2 613 052). Glaskohlenstoff und Pyrokohlenstoff haben sich nämlich als besonders körperverträglich erwiesen, weil sie sich offenbar hinreichend inert verhalten. Die glatten Elektroden bedingen jedoch relativ hohe Polarisationsverluste. Diese Verluste lassen sich aber vermeiden, wenn die Elektroden oberflächlich aktiviert sind, d. h. durch eine Oberflächenoxidation mikroporös aufgerauht werden.

Im Skelettmuskel von Katzen implantiert zeigen derartige Kohlenstoffelektroden sehr niedrige Reizschwellenströme und -spannungen und auch im Langzeitversuch steigt die Reizschwelle kaum an. Werden dieselben Elektroden dagegen im Herzen von Hunden implantiert, so bleibt zwar die Anfangsreizenergie sehr gering, die Reizschwelle steigt aber zeitlich an. Der Unterschied erklärt sich vermutlich aus der — im Vergleich mit dem Herzmuskel — geringeren und selteneren Aktivität des Skelettmuskels. Darüber hinaus gibt es Schwierigkeiten, wenn es nicht gelingt, die Elektrode schnell im Herzen zu plazieren. Beim längeren Verweilen im Blutstrom bilden sich nämlich an der mikroporösen Oberfläche offenbar Thromben, die dann bei der endgültigen Implantation der Elektrode im Herzen verhindern, daß die Elektrodenoberfläche direkt mit dem reizbaren Gewebe in Kontakt kommt. Auf diese Weise wird eine erhöhte Anfangsreizschwelle erhalten, die dann auch in einer höheren Dauerreizschwelle zum Ausdruck kommt.

Aufgabe der Erfindung ist es, bei implantierbaren Kohlenstoffelektroden die durch den postoperativen Reizschwellenanstieg im Herzmuskel auftretenden Energieverluste möglichst weitgehend zu vermeiden und auch eine Trombenbildung an der Elektrodenoberfläche, die zu erhöhten Anfangs- und Dauerreizschwellen führt, zu verhindern.

Dies wird erfindungsgemäß dadurch erreicht, daß die Elektrodenoberfläche einen glatten Überzug aus hydrophilem ionenleitendem Kunststoff in Form eines Hydrogels aufweist und daß wenigstens die Oberfläche des Kunststoff-überzuges aus körper- bzw. blutverträglichem Material besteht.

Durch den Überzug aus hydrophilem inonenleitendem Kunststoff, d. h. aus Hydrogel, wird bei der erfindungsgemäßen implantierbaren Elektrode erreicht, daß die mikroporöse Oberfläche abgedeckt und ausgeglichen wird. Der Überzug aus einer glatten wasserhaltigen Kunststoffschicht verbessert die Verträglichkeit, verringert die Reibung mit dem Muskelgewebe und verhindert gleichzeitig eine Thrombenbildung. Auf diese Weise wird der postoperative Reizschwellenanstieg eingeschränkt und es werden niedrige Dauerreizschwellenenergie erreicht.

Wegen der geforderten Langzeitstabilität unter physiologischen Bedingungen muß für den Überzug ein Material verwendet werden, das in physiologischer Umgebung entsprechend beständig ist. Außerdem ist es vorteilhaft, wenn sich das Material in flüssiger oder gelöster Form in die Poren der Elektrodenoberfläche einbringen und dort verfestigen läßt.

Als Überzugsmaterial finden bei der erfindungsgemäßen implantierbaren Elektrode, wie bereits ausgeführt, Hydrogele Verwendung. Unter Hydrogelen werden dabei Gele verstanden, die aus hydrophilen, wasserhaltigen Polymeren (Kunststoffen) bestehen. Die Überzüge werden vorteilhaft durch Polymerisation der genannten Materialien in der Porenstruktur der Elektrode hergestellt.

Ein bevorzugt verwendetes Hydrogel wird durch Polymerisation aus 2-Hydroxyäthyl-methacrylat als Kettenbildner und etwa 5 Gew.-% Glykoldimethacrylat als Brückenbildern (zum Vernetzen) mit einem üblichen Initiator, beispielsweise Ammoniumpersulfat oder Benzoylperoxid hergestellt. Als Lösungsmittel kann eine Glykol-Wasser-Mischung oder Dimethylformamid verwendet werden.

Prinzipiell sind aber alle hydrophilen Monomeren zur Polymerisation geeignet; sie können auch in Mischungen untereinander und mit hydrophoben Monomeren Anwendung finden. Die Lösungsmittelmenge richtet sich nach dem gewünschten Quellungsgrad bzw. dem angestrebten Wassergehalt und der elektrolytischen Leitfähigkeit des polymerisierten Kunststoffes. Der Wassergehalt wird im allgemeinen zwischen 30 und 90% gewählt. Ein hoher Wassergehalt ist zweckmäßig, um im gequollenen Polymeren eine hohe Ionenbeweglichkeit zu erzielen. Andererseits ist für die mechanische Festigkeit ein geringerer Quellungsgrad vorteilhaft. Als besonders vorteilhaft hat sich deshalb ein Wassergehalt von etwa 40 bis 50% erwiesen.

Neben den genannten hydrophilen Monomeren können zur Herstellung des Elektrodenüberzuges auch andere, dem Fachmann an sich bekannte Monomere Verwendung finden. So können beispielsweise bei der erfindungsgemäßen Elektrode auch Hydrogele auf der Basis von Glycerinmethacrylat eingesetzt werden. Zur Verbesserung der Körper- und Blutverträglichkeit der Elektrode — in an sich bekannter Weise — Heparin fixiert sein; es kann aber auch Heparin in den Kunststoff eingearbeitet werden.

Obwohl für die Verwendung bei der erfindungsgemäßen Elektrode hauptvalenzverkettete und vernetzte hydrophile Polymere den Vorzug erhalten, und zwar wegen ihrer Beständigkeit gegenüber der Körperflüssigkeit, sind prinzipiell auch andere hydrophile Polymere einsetzbar. Beispielhaft seien hier die hydrophilen Polyurethane erwähnt, die sich wegen ihrer guten Körper- und Blutverträglichkeit anbieten.

Die erfindungsgemäße Elektrode kann die Form einer massiven Kohleelektrode aufweisen, vorteilhaft können jedoch auch Kohlefaserbündel sowie Kohlefilze oder Kohlegewebe und Schaumkohlenstoff Verwendung finden; derartige Materialien werden dann mit Kunststoff überzogen bzw. getränkt. Als Kohlematerial dient bei der erfindungsgemäßen Elektrode vorzugsweise Glaskohlenstoff oder Pyrokohlenstoff, wobei wenigstens die Oberfläche des Elektrodenkopfes aus derartigem Material besteht. Vorteilhaft sind die Oberflächen derartiger Elektroden aktiviert, d. h. mit einer mikroporösen Struktur versehen; der Porendurchmesser liegt dabei im Bereich von etwa 0,5 nm (5 Å). Die Aktivierung, d. h. die Aufrauhung der Oberfläche, kann vorteilhaft durch eine Temperung an der Luft bei ca. 500° C erfolgen.

Erfindungsgemäße implantierbare Elektroden aus aktiviertem Glas- oder Pyrokohlenstoff, die mit einem Kunststoffüberzug versehen sind, zeichnen sich in mehrfacher Hinsicht aus:

- Vermeidung von faradayschen Elektrodenprozessen und postoperativem Reizschwellenanstieg durch Verwendung körperverträglicher Kohle als Elektrodenmaterial
- Erniedrigung von Energieverlusten aufgrund von Elektrodenpolarisation durch Aufrauhung der Elektrodenoberfläche
- Verhinderung von Thrombenbildung an der rauhen Elektrodenoberfläche durch den Kunststoffüberzug; eine Thrombenbildung erfolgt insbesondere bei der intravenösen Einführung der Elektrode in das Herz, wobei dann die thrombotischen Ablagerungen den direkten und gleichmäßigen Kontakt der Elektrode mit dem reizbaren Herzmuskelgewebe behindern.

Durch die erfindungsgemäße Elektrode läßt sich eine beachtliche Reduzierung der Reizenergie erreichen. Damit wird bei gleicher Kapazität der Energiequelle, insbesondere derjenigen eines Herzschrittmachers, eine längere Lebensdauer erreicht bzw. eine Verkleinerung von Batterie und Herzschrittmacher ermöglicht. Kleine Herzschrittmacher bringen aber für den Patienten eine Verbesserung und erleichtern den chirurgischen Eingriff. Außer als Reizelektrode für Herzschrittmacher kann die erfindungsgemäße Elektrode auch als Stimulationselektrode zur Muskel- und Nervenreizung verwendet werden.

Anhand von Ausführungsbeispielen soll die Erfindung noch näher erläutert werden.

### Beispiel 1
### (Vergleichsbeispiel)

Eine halbkugelförmige Glaskohlenstoffelektrode (Fläche: 0,09 cm²) wird eine halbe Stunde lang bei ca. 500°C an der Luft getempert und dadurch aktiviert: Dabei bildet sich eine dünne, mikroporöse Oberflächenschicht von ca. 50 μm Dicke; der Rauhigkeitsfaktor beträgt ca. 1000. Die Kapazität einer derartigen Elektrode erreicht 480 μF. Zur Kontaktierung wird ein zylinderförmiger Ansatz der Elektrode mit einer Elgiloy-Wendel mittels eines silberhaltigen leitenden Epoxidharzes verklebt und die Wendel zur Isolierung mit einem Silikonschlauch überzogen. Nach einer Behandlung mit gasförmigem Äthylenoxid bei Raumtemperatur zur Sterilisation wird die Elektrode im Herzen eines Hundes implantiert und die Langzeitreizschwelle mit einem Varioschrittmacher (Siemens-Elema AB) verfolgt. Der Varioschrittmacher ändert beim Auflegen eines Permanentmagneten seine Spannung stufenweise in 15 Schritten um je ein Fünfzehntel der Maximalspannung. Aus der Zahl der wirksamen Impulse, die sich am EKG erkennen läßt, wird die Reizschwelle ermittelt: Sie ergibt sich zu 525 mV. Die Anfangsstromreizschwelle beträgt 0,18 mA.

Bei einem anderen Implantationsversuch gelang es anfangs nicht, die Elektrode in das Herz einzuführen; sie verblieb deshalb zunächst ca. 30 Minuten in der Vene, ehe sie die endgültige Position im Herzen erreicht hatte. Die Anfangsstromreizschwelle betrug in diesem Fall 0,38 mA und die Langzeitreizschwelle stieg auf 1,05 V.

### Beispiel 2

Eine Glaskohlenstoffelektrode, die — wie in Beispiel 1 — durch Tempern an Luft aktiviert und dann kontaktiert und isoliert worden war, wird in einen mit Katalysator versetzten, handelsüblichen hydrophilen Schiffslack (Hydrogel auf der Basis von 2-Hydroxyäthyl-methacrylat; Chromsalz als Katalysator) getaucht und anschließend getrocknet. Die Elektrode wird dann in eine Kochsalzlösung eingebracht und in einer abgeschmolzenen Ampulle zur Sterilisation 20 Minuten auf 160°C erhitzt. Nach der Implantation weist sie eine Langzeitreizschwelle von 350 mV auf.

### Beispiel 3

Eine wie in Beispiel 1 aktivierte, kontaktierte und isolierte Elektrode wird unter Sauerstoffausschluß in 1 ml einer Mischung von 4,4 m Wasser, 13 ml 2-Hydroxyäthyl-methacrylat, 0,33 ml Tetraäthylenglykoldimethacrylat und 3 ml einer 5%igen Lösung von Kaliumperoxidisulfat eingebracht, auf 80°C erhitzt und bei beginnender Polymerisation herausgenommen und über der Lösung weiterhin auf einer Temperatur von 80°C gehalten. Die Elektrode wird dann 3 Stunden in Äthylenoxidgas sterilisiert und anschließend implantiert. Obwohl dabei diese Elektrode mit einem Hydrogel-Überzug bewußt 5 min frei im Blut der Herzkammer liegengelassen wurde, ehe sie in die Herzspitze vorgeschoben wurde, blieb die Anfangsstromreizschwelle bei 0,21 mA und die Dauerreizschwellenspannung unter 350 mV.

### Beispiel 4

Ein Kohlefaserbündel von etwa 2 mm Durchmesser wird an einem Ende auf eine Länge von 0,5 cm vergoldet, mit einer Elgiloy-Wendel umgeben und damit verpunktet. Das Faserbündel wird dann 1 cm hinter der vergoldeten Stelle abgeschnitten und zur Aktivierung am freien Ende 2 Minuten in auf ca. 330°C erhitzte konzentrierte Schwefelsäure getaucht. Die anhaftende Schwefelsäure wird gründlich mit destilliertem Wasser ausgewaschen und dann wird die Elektrode zur Neutralisation in eine Phosphatpufferlösung mit einem pH-Wert von 7,4 eingetaucht. Die Elgiloy-Wendel und das Kohlefaserbündel werden anschließend mit einem passenden Silikonschlauch soweit überzogen, daß noch 2,5 mm des Faserbündels herausragen. Das freie Ende des Faserbündels wird dann in 1 ml einer Mischung von 4,4 ml Wasser, 0,1 g Heparin als Natriumsalz, 13 ml 2-Hydroxyäthyl-methacrylat, 0,33 ml Tetraäthylenglykoldimethacrylat und 3 ml einer 5%igen Lösung von Kaliumperoxidisulfat getaucht, für 5 min auf 80°C erhitzt, aus der Mischung herausgenommen und soweit abgestreift, daß die Faserenden gerade noch knapp mit Kunststoff bedeckt sind. Die Polymerisation wird dann in einem geschlossenen Gefäß über der entsprechenden Mischung in 5 Stunden bei 80°C zu Ende geführt. Anschließend wird die Elektrode mit gasförmigem Äthylenoxid sterilisiert und in das Herz eines Hundes eingeführt. Die Langzeitspannungsreizschwelle bleibt unter 350 mV.

### Patentansprüche

1. Implantierbare Kohlenstoffelektrode, insbesondere Reizelektrode, dadurch gekennzeichnet, daß die Elektrodenoberfläche einen glatten Überzug aus hydrophilem ionenleitendem Kunststoff in Form eines Hydrogels aufweist und daß wenigstens die Oberfläche des Kunststoffüberzuges aus körper- bzw. blutverträglichem Material besteht.

2. Implantierbare Kohlenstoffelektrode nach Anspruch 1, dadurch gekennzeichnet, daß wenigstens die Oberfläche des Elektrodenkopfes aus Glaskohlenstoff oder Pyrokohlenstoff besteht.

3. Implantierbare Kohlenstoffelektrode nach

**0 009 727**

Anspruch 2, dadurch gekennzeichnet, daß der Glas- bzw. Pyrokohlenstoff oberflächlich aktiviert ist.

4. Implantierbare Kohlenstoffelektrode nach einem der Ansprüche 1 bis 3, dadurch gekennzeichnet, daß sie ein Kohlenstoff-Faserbündel enthält.

## Claims

1. An implantable carbon electrode, in particular a stimulation electrode, characterised in that the electrode surface has a smooth coating which consists of a hydrophilic ion-conducting synthetic resin in the form of a hydrogel, and that at least the surface of the synthetic resin coating consists of a material which is compatible with the body and blood.

2. An implantable carbon electrode as claimed in claim 1, characterised in that, at least the surface of the electrode head consists of vitreous carbon or pyrocarbon.

3. An implantable carbon electrode as claimed in claim 2, characterised in that the vitreous carbon or pyrocarbon is superficially activated.

4. An implantable carbon electrode as claimed in one of claims 1 to 3, characterised in that said electrode contains a carbon fibre bundle.

## Revendications

1. Electrode implantable en carbone, notamment électrode d'excitation, caractérisée en ce que la surface de l'électrode présente un revêtement lisse en une matière plastique hydrophile et conduisant les ions ayant la forme d'un hydrogel et au moins la surface du revêtement en matière plastique est en une matière compatible avec l'organisme et avec le sang.

2. Electrode implantable en carbone suivant la revendication 1, caractérisée en ce qu'au moins la surface de la tête à électrodes est en carbone vitreux ou en carbone pyrolytique.

3. Electrode implantable en carbone suivant la revendication 2, caractérisée en ce que le carbone vitreux ou pyrolytique est activé en surface.

4. Electrode implantable en carbone suivant l'une des revendications 1 à 3, caractérisée en ce qu'elle contient un faisceau de fibres de carbone.